# EUROPEAN PATENT SPECIFICATION

(11) **EP 4 023 332 B1**
(45) Date of publication and mention of the grant of the patent: **11.10.2023**
(21) Application number: 20905470.9
(22) Date of filing: 12.08.2020
(51) Int. Cl.: B01J 23/63, C07C 5/333, C07C 11/06, B01J 37/02

(54) **CATALYST FOR PREPARING PROPYLENE BY PROPANE DEHYDROGENATION, PREPARATION METHOD THEREFOR, AND USE THEREOF**
KATALYSATOR ZUR HERSTELLUNG VON PROPYLEN DURCH PROPANDEHYDRIERUNG, VERFAHREN ZU SEINER HERSTELLUNG UND SEINE VERWENDUNG
CATALYSEUR DE PRÉPARATION DE PROPYLÈNE PAR DÉSHYDROGÉNATION DE PROPANE, PROCÉDÉ DE PRÉPARATION ET UTILISATION ASSOCIÉS

(30) Priority: 26.12.2019 CN 201911370195
(43) Date of publication of application: 06.07.2022
(73) Proprietor: Rezel Catalysts Co., Ltd., Zhoushan, Zhejiang 316200 (CN)
(72) Inventor: ZHUO, Runsheng, Wutongqiao District Leshan, Sichuan 614000 (CN); ZHANG, Chen, Wutongqiao District Leshan, Sichuan 614000 (CN); ZHANG, Ping, Wutongqiao District Leshan, Sichuan 614000 (CN); YANG, Guangyou, Wutongqiao District Leshan, Sichuan 614000 (CN); LIU, Xinsheng, Wutongqiao District Leshan, Sichuan 614000 (CN)
(74) Representative: Bayramoglu et al.
(86) International application number: PCT/CN2020/108566
(87) International publication number: WO 2021/128867

(56) References cited:
- WO-A1-2018/108443
- WO-A2-2007/033934
- WO-A2-2007/033934
- CN-A- 102 775 262
- CN-A- 103 394 349
- CN-A- 104 072 325
- CN-A- 104 072 325
- CN-A- 104 084 198
- CN-A- 104 084 218
- CN-A- 104 148 069
- CN-A- 108 295 846
- CN-A- 109 126 788
- CN-A- 110 237 849
- CN-A- 111 085 199
- US-A- 4 762 960
- US-B2- 6 756 515

## Description

### Technical Field

The invention relates to the field of industrial catalyst preparation, in particular to a catalyst for propane dehydrogenation to propylene and a preparation method and application thereof.

### Background

Propylene is one of the important raw materials for the production of acrolein, polypropylene, acetone, polyacrylonitrile and propylene oxide. The traditional propylene production process is fluid catalytic cracking and steam cracking of naphtha and light diesel oil. With the rapid consumption of fossil energy, the traditional propylene production method cannot meet the increasing demand for propylene. Therefore, new propylene production processes have been developed, such as propane dehydrogenation (PDH), methanol to olefins (MTO) and Fischer-Tropsch synthesis (FTO). Among them, PDH process is specialized in producing propylene rather than mixed olefins. The plant is easy to be expanded to large-scale, with fewer reaction by-products, easy separation of reactants and products, and low cost. In addition, the rapid development of fracturing technology makes it possible to exploit and utilize shale gas in large scale. Propane is the main component of shale gas, which will significantly reduce the price of propane and give more prominence to the advantages of PDH process.

Up to now, PDH process includes direct PDH process without oxidant: Catofin (Lummus, CrOx based catalyst), Oleflex (UOP, Pt based catalyst) and oxidized PDH process with O₂: Star (Thyssen Krupp, Pt supported on calcium and zinc aluminates). Since the propylene selectivity of direct PDH is higher than that of oxidized PDH, direct PDH has been mainly industrialized. Direct PDH is a highly endothermic reaction with thermodynamic limitation and the activation of propane C-H bond is the decisive step which determines the catalytic performance of PDH. The C-H bond of propane is very stable, so a higher reaction temperature (550-700°C) is needed to achieve C-H bond fracture. However, at high temperature C-C bond breaking is more favorable than C-H bond breaking, and propylene is more difficult to desorb than propane, so it is prone to cracking, deep dehydrogenation or polymerization reactions, resulting in low selectivity and coking.

Compared with CrOx-based catalysts, Pt based catalysts have the advantages of environmental protection and high stability. However, the supported Pt catalyst without promoter and unmodified support has no selectivity for propylene, which is easy to form heavy carbon deposition to cover the active sites, resulting in rapid deactivation of the catalyst. Therefore, it is necessary to add additives to improve propylene selectivity. At present, the research is mainly made on Pt-Sn based catalysts.

For example, patent CN108722468B discloses a propane dehydrogenation catalyst with spherical montmorillonite mesoporous composite as support, Pt as active component, Sn and Na as promoters. With the help of novel support with three peak por size distribution and appropriate Pt/Sn ratio, the catalyst shows good catalytic performance and stability.

Patent CN109529911A discloses a propane dehydrogenation catalyst with SBA-16 mesoporous silicon as support and Pt and Sn as active components. SBA-16 is prepared with cetyltrimethylammonium bromide and triblock polymer as template. The catalyst has uniform framework structure and pore distribution, high catalytic performance and stability for propane dehydrogenation.

CN104072325A discloses a propane dehydrogenation catalyst comprising platinum, tin, lithium and cerium on alumina beads.

WO2007/033934A2 discloses a propane dehydrogenation catalyst comprising platinum, tin, potassium, cesium and lanthanum on a support.

However, Pt-Sn catalyst is still easy to be deactivated because of the following reasons: 1) the active component Pt particles are sintered and covered by carbon deposition; 2) partial reduction of Sn in propane dehydrogenation atmosphere results in Pt-Sn alloy which poisons Pt; 3) the specific surface area, pore volume and pore diameter change of Al₂O₃ occurs under the reaction condition; 4) Pt-Sn loading cannot completely eliminate the inherent acid sites of Al₂O₃ which cause serious deep dehydrogenation, cracking and coking. Therefore, it is necessary to further improve the Pt-Sn catalyst by introducing other components, enhancing the interaction between Pt and support and promoter, inhibiting Pt sintering and Sn segregating resulting in irreversible poisoning of Pt, and reducing the acidity of Al₂O₃.

The introduction of low acidic oxides into the carrier can reduce the acidity of the carrier, improve the "Pt-carrier" interaction, and limit the segregation and reduction of Sn. Generally, the addition of alkali metal elements (Li, Na, K) can reduce the acidity of the carrier, and causes part of its activity loss. Rare earth metal elements are potential excellent promoters: for example, Y-modified ZrO₂ support can significantly improve the dispersion and anti-sintering ability of Pt/YSZ (fuel, 2010, 89, 2244-2251) and Pt (Rh, Pd)/YSZ catalysts. Y modified Al₂O₃ can also improve the interactions between Pd and carrier in Pd / Ce-Zr-Y/Al₂O₃ catalyst, and inhibit other components in the catalyst causing Pd poisoning. La can also play a similar role as reported in Fuel. Process. Technol., 2013, 111, 94-104.

To sum up, although Pt-Sn based catalysts are the main catalysts for propane dehydrogenation to propylene, and Sn can improve the propylene selectivity and propylene yield of the catalysts, the support acidity is still strong, the instability of Pt particles and the alloying of Sn to Pt are still easy to lead to the deactivation of Pt-Sn catalysts. Therefore, the problem needs to be resolved.

### Summary

In order to solve the above technical problems, the invention provides a catalyst for propane dehydrogenation to propylene, a preparation method and application thereof. The purpose of this invention is to modify and improve the Pt-Sn based catalyst, and improve its catalytic performance and stability.

The invention is realized by the following technical scheme:
A catalyst for propane dehydrogenation to propylene comprises a carrier, an active component supported on the carrier, a first promoter, a second promoter and a third promoter;
The carrier is alumina ball; the active component is noble metal Pt; the rare earth metal elements of the first promoter is Y; the second promoter is Sn; the alkali metal elements of the third promoter is K.

As for the content of each component in the catalyst, the mass fraction of the carrier is 80%-99.6%, preferably 96.2%-98.7%, taking the total mass of the catalyst as the standard;
The mass fraction of the active component Pt is 0.1%-5%, preferably 0.2%-0.5%;
The content of rare earth metal element Y in the first promoter is 0. 1%-5%, preferably 0.5%-2%;
The mass fraction of Sn in the second promoter is 0.1%-5%, preferably 0.1%-0.3%;
The mass fraction of the third promoter K is 0.1%-5%, preferably 0.5%-1%.

In a preferred scheme of the invention, the mass fraction of support is 96.2%-98.7%, the mass fraction of Pt is 0.2%-0.5%, the mass fraction of rare eart element Y is 0.5%-2%, the mass fraction of Sn is 0. 1%-0.3%, and the mass fraction of K is 0.5%-1%.

A preparation method of a catalyst for propane dehydrogenation to propylene includes the following steps:
1) Pt precursor, Sn precursor and competitive adsorption agent are dissolved in water to form impregnation solution A;
2) The alumina ball carrier is impregnated in the impregnation solution A, and then dried and calcined;
3) The rare earth metal precursor and K precursor are dissolved in water to form impregnation solution B;
4) The catalyst for propane dehydrogenation is prepared by impregnating the carrier in impregnation solution B, drying and calcining after impregnation.

Furthermore, in step 1), Pt precursor is chloroplatinic acid and Sn precursor is SnCl₂;
Furthermore, the competitive adsorption agent in step 1) is one of the following inorganic and organic acid: concentrated hydrochloric acid, concentrated nitric acid, oxalic acid and citric acid, and concentrated hydrochloric acid is preferred;
Furthermore, the amount of competitive adsorption agent in step 1) is 7-9% of impregnation solution A;
Furthermore, the rare earth metal precursors in step 3) are soluble salts, preferably YCl;
Furthermore, the K precursor in step 3) is soluble potassium salt, and KNO₃ is preferred;
Furthermore, in step 2) and step 4), the impregnated alumina balls are dried in vacuum at 80-120°C for 6-10 h and calcined at 550-650°C for 3-5 h to obtain the catalyst for propane dehydrogenation to olefins.

Use of the catalyst of the present application in propane dehydrogenation to propylene: specifically, the reaction conditions of propane dehydrogenation to propylene are as follows: the reaction is carried out in a quartz tube fixed bed reactor, the reaction temperature is 550°C-650°C, the total space velocity is 1 h⁻¹-5 h⁻¹, and the hydrogen/propane flow ratio is 1/4-1/1.

The invention uses rare earth metal element Y, as the first promoter to modify and improve the Pt-Sn based catalyst, so as to reduce the support acidity, inhibit Pt sintering and Sn segregation, and Sn alloying of Pt, and improve the catalytic performance and stability of the catalyst.

Compared with the prior art, the invention has the following advantages and beneficial effects:
1. The catalyst for propane dehydrogenation to propylene is modified and improved by using rare earth metal element as the first promoter, which has high catalytic performance and stability;
2. The preparation method of the catalyst for propane dehydrogenation to propylene reduces the acidity of the support, inhibits Pt sintering and Sn segregation, and Sn alloying of Pt, and improves the catalytic performance and stability of the catalyst.

### Detailed Description of the Embodiments

In order to make the purpose, the technical scheme and the advantages of the invention clearer, the present invention is further described in detail in combination with the embodiment. The schematic implementation and description of the invention are only used to explain the invention, not as the limitation of the invention.

### Example 1

In this embodiment, Catalyst 1, Pt-Sn-K-Y/Al₂O₃ is prepared, and its catalytic performance for propane dehydrogenation to propylene is tested.

Preparation: 1) prepare 0.02 g/g H₂PtCl₆·6H₂O aqueous solution and 0.04 g/g SnCl₂·2H₂O aqueous solution.
Take 4.075 g and 0.729 g H₂PtCl₆·6H₂O solution and SnCl₂·2H₂O solution respectively and put into a glass beaker, then add concentrated hydrochloric acid and deionized water, so that the volume of Pt-Sn impregnation solution is equal to the total volume of water absorbed by 10 g Al₂O₃ carrier, and the mass of concentrated hydrochloric acid is 8.3% of the mass of impregnation solution. After stirring evenly, impregnate 10 g alumina ball carrier with the solution. After soaking for 4 h, vacuum drying at 100°C for 8 h and then calcine at 600°C for 4 h;
2) Prepare KNO₃ aqueous solution with mass concentration of 0.07 g/g and YCl₃ aqueous solution with mass concentration of 0.312 g/g respectively.

Take 3.023 g and 0.722 g KNO₃ solution and YCl₃ solution and put into a glass beaker and then add deionized water to make the volume of K-Y impregnation solution equal to the total volume of water absorbed by 10 g Al₂O₃ carrier. After stirring evenly, the alumina ball carrier calcined in step 1) is impregnated with the K-Y impregnation solution. The final Pt-Sn-K-Y/Al₂O₃ catalyst for propane dehydrogenation is then obtained by impregnation for 4 h, vacuum drying at 100°C for 8 h and calcination at 600°C for 4 h.

Catalyst evaluation: a quartz tube fixed bed reactor was filled with 3 g Catalyst 1, the total mass space velocity of hydrogen and propane was controlled at 3 h⁻¹, the hydrogen/propane flow ratio was 1/4, the reaction pressure was atmospheric pressure, the bed temperature was 610°C, and the reaction products were analyzed by using Shimadzu GC-2014C gas chromatography.

### Example 2 (not claimed)

In this embodiment, Catalyst 2, Pt-Sn-K-La/Al₂O₃ is prepared, and its catalytic performance for propane dehydrogenation to propylene is tested.

Preparation: 1) prepare 0.02 g/g H₂PtCl₆·6H₂O aqueous solution and 0.04 g/g SnCl₂·2H₂O aqueous solution.
Take 4.075 g and 0.729 g H₂PtCl₆·6H₂O solution and SnCl₂·2H₂O solution respectively and put into a glass beaker, then add concentrated hydrochloric acid and deionized water, so that the volume of Pt-Sn impregnation solution is equal to the total volume of water adsorbed by 10 g Al₂O₃ carrier, and the mass of concentrated hydrochloric acid is 8.3% of the mass of impregnation solution. After stirring evenly, impregnate 10g alumina ball carrier with this solution. Then soaking for 4 h, vacuum drying at 100°C for 8 h and calcining at 600°C for 4 h;
2) Prepare KNO₃ aqueous solution with mass concentration of 0.07 g/g and LaCl₃ aqueous solution with mass concentration of 0.271 g/g respectively.

Take 3.023 g and 0.667 g KNO₃ solution and LaCl₃ solution respectively and put into a glass beaker, and then add deionized water to make the volume of K-La impregnation solution equal to the total volume of water absorbed by 10 g Al₂O₃ carrier. After stirring evenly, the alumina ball carrier calcined in step 1) is impregnated with this solution. The final Pt-Sn-K-La/Al₂O₃ catalyst for propane dehydrogenation is obtained by impregnation for 4h, vacuum drying at 100°C for 8 h and calcination at 600°C for 4 h.

Catalyst evaluation: a quartz tube fixed bed reactor was filled with 3 g Catalyst 2, the total mass space velocity of hydrogen and propane was controlled to be 3 h⁻¹, the hydrogen/propane flow ratio was 1/4, the reaction pressure was atmospheric pressure, the bed temperature was 610°C, and the reaction products were analyzed by using Shimadzu GC-2014C gas chromatography.

### Example 3 (not claimed)

In this embodiment, Catalyst 3, Pt-Sn-K-Ce/Al₂O₃ is prepared, and its catalytic performance for propane dehydrogenation to propylene is tested.
Preparation: 1) prepare 0.02 g/g H₂PtCl₆·6H₂O aqueous solution and 0.04 g/g SnCl₂·2H₂O aqueous solution. Take 4.075 g and 0.729 g H₂PtCl₆·6H₂O solution and SnCl₂·2H₂O solution respectively and put into a glass beaker, then add concentrated hydrochloric acid and deionized water, so that the volume of Pt-Sn impregnation solution is equal to the total volume of water absorbed by 10 g Al₂O₃ carrier, and the mass of concentrated hydrochloric acid is 8.3% of the mass of impregnation solution. After stirring evenly, impregnate 10 g alumina ball carrier with this solution. Then soaking for 4 h, vacuum drying at 100°C for 8 h and calcining at 600°C for 4 h;
2) Prepare KNO₃ aqueous solution with mass concentration of 0.07 g/g and Ce(NO₃)₃·6H₂O aqueous solution with mass concentration of 0.2 g/g respectively.

Take 3.023 g and 1.585 g KNO₃ solution and Ce(NO₃)₃·6H₂O solution respectively and put into a glass beaker, and then add deionized water to make the volume of K-Ce impregnation solution equal to the total volume of water absorbed by 10 g Al₂O₃ carrier. After stirring evenly, the alumina ball carrier calcined in step 1) is impregnated with this solution. The final Pt-Sn-K-Ce/Al₂O₃ catalyst for propane dehydrogenation was obtained by impregnation for 4h, vacuum drying at 100°C for 8 h and calcination at 600°C for 4 h.

Catalyst evaluation: a quartz tube fixed bed reactor was filled with 3 g Catalyst 3, the total mass space velocity of hydrogen and propane was controlled to be 3 h⁻¹, the hydrogen / propane flow ratio was 1/4, the reaction pressure was atmospheric pressure, the bed temperature was 610°C, and the reaction products were analyzed by using Shimadzu GC-2014C gas chromatography.

### Example 4 (not claimed)

In this embodiment, Catalyst 4, Pt-Sn-K-Pr/Al₂O₃ is prepared, and its catalytic performance for propane dehydrogenation to propylene is tested.

Preparation: 1) prepare 0.02 g/g H₂PtCl₆·6H₂O aqueous solution and 0.04 g/g SnCl₂·2H₂O aqueous solution.
Take 4.075 g and 0.729 g H₂PtCl₆·6H₂O solution and SnCl₂·2H₂O solution respectively and put into a glass beaker, then add concentrated hydrochloric acid and deionized water, so that the volume of Pt-Sn impregnation solution is equal to the total volume of water absorbed by 10 g Al₂O₃ carrier, and the mass of concentrated hydrochloric acid is 8.3% of the mass of impregnation solution. After stirring evenly, impregnate 10g alumina ball carrier with this solution. Then soaking for 4 h, vacuum drying at 100°C for 8 h and calcining at 600°C for 4 h;
2) Prepare KNO₃ aqueous solution with mass concentration of 0.07 g/g and Pr(NO₃)₃·6H₂O aqueous solution with mass concentration of 0.2 g/g respectively.

Take the KNO₃ solution and Pr(NO₃)₃·6H₂O solution 3.023 g and 1.579 g respectively, then add deionized water to make the volume of K-Pr impregnation solution equal to the total volume of water absorbed by 10 g Al₂O₃ carrier. After stirring evenly, the alumina ball carrier calcined in step 1) is impregnated with this solution. The final Pt-Sn-K-Pr/Al₂O₃ catalyst for propane dehydrogenation was obtained by impregnation for 4h, vacuum drying at 100°C for 8h and calcination at 600°C for 4h.

Catalyst evaluation: a quartz tube fixed bed reactor was filled with 3 g Catalyst 4, the total mass space velocity of hydrogen and propane was controlled to be 3 h⁻¹, the hydrogen / propane flow ratio was 1/4, the reaction pressure was atmospheric pressure, the bed temperature was 610°C, and the reaction products were analyzed by using Shimadzu GC-2014C gas chromatography.

### Example 5 (not claimed)

In this embodiment, Catalyst 4, Pt-Sn-K-Nd/Al₂O₃ is prepared, and its catalytic performance for propane dehydrogenation to propylene is tested.

Preparation: 1) prepare 0.02 g/g H₂PtCl₆·6H₂O aqueous solution and 0.04 g/g SnCl₂·2H₂O aqueous solution.
Take 4.075 g and 0.729 g H₂PtCl₆·6H₂O solution and SnCl₂·2H₂O solution respectively and put into a glass beaker, then add concentrated hydrochloric acid and deionized water, so that the volume of Pt-Sn impregnation solution is equal to the total volume of water absorbed by 10 g Al₂O₃ carrier, and the mass of concentrated hydrochloric acid is 8.3% of the mass of impregnation solution. After stirring evenly, impregnate 10g alumina ball carrier with this solution. Then soaking for 4 h, vacuum drying at 100°C for 8 h and calcining at 600°C for 4 h;
2) Prepare KNO₃ aqueous solution with mass concentration of 0.07g/g and Nd(NO₃)₃·6H₂O aqueous solution with mass concentration of 0.2g/g.

Take 3.023 g and 1.555 g of KNO₃ solution and Nd(NO₃)₃·6H₂O solution and put into a glass beaker, and then add deionized water to make the volume of K-Pr impregnation solution equal to the total volume of water absorbed by 10 g Al₂O₃ carrier. After stirring evenly, the alumina ball carrier calcined in step 1) is impregnated with this solution. The final Pt-Sn-K-Nd/Al₂O₃ catalyst for propane dehydrogenation was obtained by impregnation for 4h, vacuum drying at 100°C for 8h and calcination at 600°C for 4h.

Catalyst evaluation: a quartz tube fixed bed reactor was filled with 3 g Catalyst 5, the total mass space velocity of hydrogen and propane was controlled to be 3 h⁻¹, the hydrogen / propane flow ratio was 1/4, the reaction pressure was atmospheric pressure, the bed temperature was 610°C, and the reaction products were analyzed by using Shimadzu GC-2014C gas chromatography.

### Comparative example 1

In this comparative embodiment, Catalyst 6, Pt-Sn-K/Al₂O₃ is prepared, and its catalytic performance for propane dehydrogenation to propylene is tested. The catalyst does not contain any rare earth elements as the first promoter.

Preparation: 1) prepare 0.02 g/g H₂PtCl₆·6H₂O aqueous solution and 0.04 g/g SnCl₂·2H₂O aqueous solution.
Take 4.075 g and 0.729 g H₂PtCl₆·6H₂O solution and SnCl₂·2H₂O solution respectively and put into a glass beaker, then add concentrated hydrochloric acid and deionized water, so that the volume of Pt-Sn impregnation solution is equal to the total volume of water adsorbed by 10 g Al₂O₃ carrier, and the mass of concentrated hydrochloric acid is 8.3% of the mass of impregnation solution. After stirring evenly, impregnate 10 g alumina ball carrier with this solution. Then soaking for 4 h, vacuum drying at 100°C for 8 h and calcining at 600°C for 4 h;
2) Prepare KNO₃ aqueous solution with mass concentration of 0.07 g/g.

Take 3.023 g KNO₃ solution and put into a glass beaker, and then add deionized water to make the volume of K impregnation solution equal to the total volume of water absorbed by 10 g Al₂O₃ carrier. After stirring thoroughly, the alumina ball carrier calcined in step 1) is impregnated with this solution. The final Pt-Sn-K/Al₂O₃ catalyst for propane dehydrogenation was obtained by impregnation for 4h, vacuum drying at 100°C for 8h and calcination at 600°C for 4h.

Catalyst evaluation: a quartz tube fixed bed reactor was filled with 3 g Catalyst 6, the total mass space velocity of hydrogen and propane was controlled to be 3 h⁻¹, the hydrogen/propane flow ratio was 1/4, the reaction pressure was atmospheric pressure, the bed temperature was 610°C, and the reaction products were analyzed by using Shimadzu GC-2014C gas chromatography.

The content of active components and promoters of Catalysts 1-6 is shown in Table 1; The testing results of catalytic performance of the catalysts prepared by examples 1-5 and comparative example 1 are given in Table 2.

**Table 1. Content of active component and promotors in the catalysts**

| | **Component Mass Fractions (%)** | | | | | | | |
|---|---|---|---|---|---|---|---|---|
| | **Pt** | **Sn** | **K** | **Y** | **La** | **Ce** | **Pr** | **Nd** |
| Catalyst **1** | 0.3 | 0.15 | 0.8 | 1.0 | - | - | - | - |
| Catalyst **2** | 0.3 | 0.15 | 0.8 | - | 1.0 | - | - | - |
| Catalyst **3** | 0.3 | 0.15 | 0.8 | - | - | 1.0 | - | - |
| Catalyst **4** | 0.3 | 0.15 | 0.8 | - | - | - | 1.0 | |
| Catalyst **5** | 0.3 | 0.15 | 0.8 | - | - | - | - | 1.0 |
| Catalyst **6** | 0.3 | 0.15 | 0.8 | - | - | - | - | - |

**Table 2. Catalyst catalytic performance**

| **No.** | **Catalyst** | **Initial catalyst performance ( %)** | | | **3h average Performance (%)** | | |
|---|---|---|---|---|---|---|---|
| | | **Propane Conv. (%)** | **Propylene Select. (%)** | **Propylen e Yield (%)** | **Propane Conv. (%)** | **Propylene Select. (%)** | **Propyle ne Yield (%)** |
| **Example 1** | **Pt-Sn-K-Y/Al₂O₃** | 58.10 | 74.15 | 43.08 | 44.18 | 83.04 | 36.62 |
| **Example 2** | **Pt-Sn-K-La/Al₂O₃** | 56.17 | 75.63 | 42.49 | 37.18 | 81.15 | 30.15 |
| **Example 3** | **Pt-Sn-K-Ce/Al₂O₃** | 38.89 | 74.67 | 29.04 | 24.64 | 72.75 | 17.99 |
| **Example 4** | **Pt-Sn-K-Pr/Al₂O₃** | 50.96 | 75.97 | 38.72 | 28.18 | 75.95 | 21.49 |
| **Example 5** | **Pt-Sn-K-Nd/Al₂O₃** | | | | | | |
| **Comparativ e example 1** | **Pt-Sn-K/Al₂O₃** | 47.54 | 86.51 | 41.12 | 35.75 | 81.18 | 29.09 |

Combined with the evaluation results in Tables 1 and 2, it is shown that the performance of Pt-Sn based propane dehydrogenation catalyst can be modified by adding rare earth metal elements. Compared with comparative example 1, Y can significantly improve the catalytic performance and stability of Pt-Sn based propane dehydrogenation catalyst, while La can only play a slightly improving role. Ce and Pr decrease the catalytic performance and stability of Pt-Sn based catalysts.

## Claims

1. A catalyst for propane dehydrogenation to propylene, the catalyst comprising a support, an active component and a promoter supported on the support, wherein the support is an alumina ball, the active component is Pt, and the promoter includes rare earth elements of the first promoter, tin of the second promoter and alkali metal elements of the third promoter wherein based on the total mass of the catalyst, the mass fraction of the carrier is 80%-99.6%, the mass fraction of the active component Pt is 0.1%-5%, the mass fraction of rare earth elements in the first promoter is 0. 1%-5%, the mass fraction of Sn in the second promoter is 0. 1%-5%, and the mass fraction of alkali metal element in the third promoter is 0. 1%-5%, and **characterized in that** the rare earth element of the first promoter is Y and the alkali metal element of the third promoter is K.

2. A preparation method of a catalyst for propane dehydrogenation to propylene according to any claim 1, **characterized in that** comprising the following contents:
1) dissolving Pt precursor and Sn precursor in water to form a mixed solution, and then adding competitive agent to obtain impregnation solution A;
2) impregnating the alumina ball carrier with the impregnation solution A, and then drying and calcining;
3) dissolving the rare earth metal precursor and K precursor in water to form a mixed solution which is impregnation solution B;
4) impregnating the carrier calcined in step 2) with the impregnation solution B, after the impregnation is finished, drying and calcining to obtain the propane dehydrogenation catalyst.

3. The preparation method of a catalyst for propane dehydrogenation to propylene according to claim 2, **characterized in that** the Pt precursor is chloroplatinic acid and the Sn precursor is SnCl₂.

4. The preparation method of a catalyst for propane dehydrogenation to propylene according to claim 2, **characterized in that** the competitive adsorption agent is selected from one or more of the following inorganic acid or organic acid: concentrated hydrochloric acid, concentrated nitric acid, oxalic acid and citric acid, and the amount of competitive adsorption agent is 7-9% of the mass of impregnation solution A.

5. The preparation method of a catalyst for propane dehydrogenation to propylene according to claim 2, **characterized in that** the rare earth metal precursor is a soluble salt of rare earth metal elements.

6. The preparation method of a catalyst for propane dehydrogenation to propylene according to claim 2, **characterized in that** the K precursor is soluble potassium salt.

7. The preparation method of a catalyst for propane dehydrogenation to propylene according to claim 2, **characterized in that** in steps 2) and 4), the drying adopts vacuum drying, the drying temperature is 80-120°C, and the drying time is 6-10 h; the calcination temperature is 550-650°C, and the calcination time is 3-5 h.

8. The application of a catalyst for propane dehydrogenation to propylene according to claim 1, **characterized in that** propane dehydrogenation test is carried out in a quartz tube fixed bed reactor, the dehydrogenation reaction temperature is 550°C-650°C, the mass space velocity is 1 h⁻¹-5 h⁻¹, and the hydrogen/propane flow ratio is 1/4-1/.

## Patentansprüche

1. Katalysator zur Propandehydrierung zu Propylen, wobei der Katalysator einen Träger, eine aktive Komponente und einen auf dem Träger getragenen Promotor umfasst, wobei der Träger eine Aluminiumoxidkugel ist, die aktive Komponente Pt ist und der Promotor Seltenerdelemente des ersten Promotors, Zinn des zweiten Promotors und Alkalimetallelemente des dritten Promotors umfasst, wobei, bezogen auf die Gesamtmasse des Katalysators, der Massenanteil des Trägers 80 % bis 99,6 % beträgt, der Massenanteil der aktiven Komponente Pt 0,1 % bis 5 % beträgt, der Massenanteil der Seltenerdelemente im ersten Promotor 0,1 % bis 5 % beträgt, der Massenanteil von Sn im zweiten Promotor 0,1 % bis 5 % beträgt und der Massenanteil des Alkalimetallelements im dritten Promotor 0,1 % bis 5 % beträgt, und **dadurch gekennzeichnet, dass** das Seltenerdelement des ersten Promotors Y ist und das Alkalimetallelement des dritten Promotors K ist.

2. Verfahren zur Herstellung eines Katalysators zur Propandehydrierung zu Propylen nach Anspruch 1, **gekennzeichnet durch**:
1) Lösen eines Pt-Vorläufers und eines Sn-Vorläufers in Wasser, um eine gemischte Lösung zu bilden, und dann Zugeben von Konkurrenzmittel, um eine Imprägnierlösung A zu erhalten;
2) Imprägnieren des Aluminiumoxidkugelträgers mit der Imprägnierlösung A und dann Trocknen und Kalzinieren;
3) Lösen des Seltenerdmetallvorläufers und des K-Vorläufers in Wasser, um eine gemischte Lösung zu bilden, die eine Imprägnierlösung B ist;
4) Imprägnieren des in Schritt 2) kalzinierten Trägers mit der Imprägnierlösung B, nachdem die Imprägnierung beendet ist, Trocknen und Kalzinieren, um den Propandehydrierungskatalysator zu erlangen.

3. Verfahren zur Herstellung eines Katalysators zur Propandehydrierung zu Propylen nach Anspruch 2, **dadurch gekennzeichnet, dass** der Pt-Vorläufer Chlorplatinsäure ist und der Sn-Vorläufer SnCl₂ ist.

4. Verfahren zur Herstellung eines Katalysators zur Propandehydrierung zu Propylen nach Anspruch 2, **dadurch gekennzeichnet, dass** das Konkurrenzadsorptionsmittel ausgewählt ist aus einer oder mehreren der folgenden anorganischen oder organischen Säuren: konzentrierte Salzsäure, konzentrierte Salpetersäure, Oxalsäure und Zitronensäure, und die Menge des Konkurrenzadsorptionsmittels 7 bis 9 % der Masse der Imprägnierlösung A beträgt.

5. Verfahren zur Herstellung eines Katalysators zur Propandehydrierung zu Propylen nach Anspruch 2, **dadurch gekennzeichnet, dass** der Seltenerdmetallvorläufer ein lösliches Salz von Seltenerdmetallelementen ist.

6. Verfahren zur Herstellung eines Katalysators zur Propandehydrierung zu Propylen nach Anspruch 2, **dadurch gekennzeichnet, dass** der K-Vorläufer ein lösliches Kaliumsalz ist.

7. Verfahren zur Herstellung eines Katalysators zur Propandehydrierung zu Propylen nach Anspruch 2, **dadurch gekennzeichnet, dass** in den Schritten 2) und 4) als Verfahren zum Trocknen Vakuumtrocknen gewählt ist, die Trocknungstemperatur 80 bis 120 °C beträgt und die Trocknungszeit 6 bis 10 h beträgt; die Kalzinierungstemperatur 550 bis 650 °C beträgt und die Kalzinierungszeit 3 bis 5 h beträgt.

8. Verwendung eines Katalysators zur Propandehydrierung zu Propylen nach Anspruch 1, **dadurch gekennzeichnet, dass** ein Propandehydrierungstest in einem Quarzrohr-Festbettreaktor durchgeführt wird, die Dehydrierungsreaktionstemperatur 550 °C bis 650 °C beträgt, die Massenraumgeschwindigkeit 1 h⁻¹ bis 5 h⁻¹ beträgt und das Wasserstoff/Propan-Strömungsverhältnis 1/4 bis 1/ beträgt.

## Revendications

1. Catalyseur pour la déshydrogénation de propane en propylène, le catalyseur comprenant un support, un composant actif et un promoteur supporté sur le support, le support est une boule d'alumine, le composant actif étant du Pt, et le promoteur comportant des éléments de terres rares du premier promoteur, de l'étain du deuxième promoteur et des éléments de métaux alcalins du troisième promoteur, sur la base de la masse totale du catalyseur, la fraction massique du support étant de 80% à 99,6%, la fraction massique du composant actif Pt étant de 0,1% à 5%, la fraction massique des éléments de terres rares dans le premier promoteur étant de 0,1% à 5%, la fraction massique de Sn dans le deuxième promoteur étant de 0,1% à 5% et la fraction massique de l'élément de métal alcalin dans le troisième promoteur étant de 0,1% à 5%, et **caractérisé en ce que** l'élément de terres rares de terres rares du premier promoteur est de l'Y et l'élément de métal alcalin du troisième promoteur est du K.

2. Procédé de préparation d'un catalyseur pour la déshydrogénation de propane en propylène selon la revendication 1, **caractérisé en ce qu'**il comprend les contenus suivants :
1) la dissolution du précurseur de Pt et du précurseur de Sn dans de l'eau pour former une solution mixte, puis l'ajout d'un agent compétitif pour obtenir une solution d'imprégnation A ;
2) l'imprégnation du support en boule d'alumine avec la solution d'imprégnation A, puis séchage et calcination ;
3) la dissolution du précurseur de métal de terres rares et du précurseur de K dans de l'eau pour former une solution mixte qui est la solution d'imprégnation B ;
4) l'imprégnation du support calciné à l'étape 2) avec la solution d'imprégnation B, une fois l'imprégnation terminée, séchage et calcination pour obtenir le catalyseur de déshydrogénation de propane.

3. Procédé de préparation d'un catalyseur de déshydrogénation de propane en propylène selon la revendication 2, **caractérisé en ce que** le précurseur de Pt est de l'acide chloroplatinique et le précurseur de Sn est du SnCl₂.

4. Procédé de préparation d'un catalyseur pour la déshydrogénation du propane en propylène selon la revendication 2, **caractérisé en ce que** l'agent d'adsorption compétitif est choisi parmi un ou plusieurs des acides inorganiques ou acides organiques suivants : acide chlorhydrique concentré, acide nitrique concentré, acide oxalique et acide citrique, et la quantité d'agent d'adsorption compétitif est de 7 à 9% de la masse de solution d'imprégnation A.

5. Procédé de préparation d'un catalyseur pour la déshydrogénation de propane en propylène selon la revendication 2, **caractérisé en ce que** le précurseur de métaux de terres rares est un sel soluble d'éléments de métaux de terres rares.

6. Procédé de préparation d'un catalyseur de déshydrogénation de propane en propylène selon la revendication 2, **caractérisé en ce que** le précurseur de K est un sel de potassium soluble.

7. Procédé de préparation d'un catalyseur pour la déshydrogénation du propane en propylène selon la revendication 2, **caractérisé en ce que** dans les étapes 2) et 4), le séchage adopte un séchage sous vide, la température de séchage est de 80 à 120°C et la durée de séchage est de 6 à 10 h ; la température de calcination est de 550 à 650°C et la durée de calcination est de 3 à 5 h.

8. Application d'un catalyseur pour la déshydrogénation de propane en propylène selon la revendication 1, **caractérisée en ce que** l'essai de déshydrogénation de propane est réalisé dans un réacteur à lit fixe en tube de quartz, la température de la réaction de déshydrogénation est de 550°C à 650°C, la vitesse spatiale massique est de 1 h⁻¹ à 5 h⁻¹, et le rapport de débit hydrogène/propane est de 1/4 à 1/.
